(19) 

Europäisches Patentamt
European Patent Office
Office européen des brevets

(11) **EP 4 379 049 A1**

(12) **EUROPEAN PATENT APPLICATION**
published in accordance with Art. 153(4) EPC

(43) Date of publication:
**05.06.2024 Bulletin 2024/23**

(21) Application number: **22849552.9**

(22) Date of filing: **27.07.2022**

(51) International Patent Classification (IPC):
**C12N 9/08** (2006.01)  **A23C 9/13** (2006.01)
**A23C 9/152** (2006.01)  **C12N 9/50** (2006.01)
**A23C 7/04** (2006.01)

(52) Cooperative Patent Classification (CPC):
**A23C 9/13; A23C 9/152; C12N 9/0004; C12N 9/50;**
A23C 7/04

(86) International application number:
**PCT/JP2022/028982**

(87) International publication number:
**WO 2023/008491 (02.02.2023 Gazette 2023/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.07.2021 JP 2021122089**

(71) Applicant: **Godo Shusei Co., Ltd.
Matsudo-shi, Chiba 271-0064 (JP)**

(72) Inventors:
• **IIZAKA MORIE, Kyoko
Matsudo-shi, Chiba 271-0064 (JP)**

• **ARAYA, Naho
Matsudo-shi, Chiba 271-0064 (JP)**
• **SHIOTA, Kazuma
Matsudo-shi, Chiba 271-0064 (JP)**

(74) Representative: **Wächtershäuser & Hartz
Patentanwaltspartnerschaft mbB
Weinstraße 8
80333 München (DE)**

Remarks:
The complete document including Reference
Table(s) and the Sequence Listing(s) can be
downloaded from the EPO website

(54) **ENZYME-CONTAINING COMPOSITION, METHOD FOR PRODUCING MILK AND METHOD FOR PRODUCING FERMENTED MILK**

(57) To provide an enzyme-containing composition, less likely to generate curds by the action of peroxidase even when the peroxidase is reacted with the milk for a long time, a method for producing milk, and a method for producing fermented milk. An enzyme-containing composition, in which a ratio of the protease activity to the peroxidase activity is 10 or less.

EP 4 379 049 A1

**Description**

Technical Field

The present invention relates to a composition containing an enzyme having peroxidase activity, and a method for manufacturing milk and a method for manufacturing fermented milk each using the enzyme-containing composition.

Background Art

Processes for manufacturing milk generally involve the followings:

(1) milking livestock such as a cow;
(2) collecting raw milk and transporting it to a factory;
(3) conducting acceptance inspection at the factory;
(4) cooling the raw milk;
(5) storing the cooled raw milk;
(6) removing contaminants from the raw milk and preheating the milk;
(7) heat-sterilizing the preheated milk;
(8) cooling and then storing the heat-sterilized milk;
(9) performing filling and packaging; and
(10) shipping the product after inspection.

Consumers can purchase the milk that has undergone the above processes at a retail store or the like.

It is known that milk and yoghurt in a filling package contain a peroxide (for example, see Patent Literature 1). This Patent Literature 1 states that this peroxide can be reduced by utilizing peroxidase.

Citation List

Patent Literature

Patent Literature 1: JP-B-08-004453

Summary of Invention

Technical Problem

Patent Literature 1 discloses that peroxidase is added to commercially available milk and yoghurt and an enzyme reaction is performed at room temperature or 25°C for 15 minutes (for example, Examples 1 and 2). However, Patent Literature 1 does not describe anything about the addition of peroxidase to a commercially available dairy product and a long-time enzyme reaction.

Since addition of peroxidase to a dairy product and subsequent deactivation of the added peroxidase may influence the taste and so on of the dairy product, it is preferable not to perform additional processing. As the present inventors added peroxidase (an enzyme-containing composition before purification which will be described in Example 3 later) to commercially available milk and performed a long-time enzyme reaction, it was observed that the commercially available milk turned into curds, which was an unexpected phenomenon.

The present invention aims to provide an enzyme-containing composition that is less likely to generate curds even when peroxidase is reacted with the milk for a long time, a method for manufacturing milk, and a method for manufacturing fermented milk.

Solution

The present inventors have conducted various studies to solve the above problems. As a result, the present inventors found that milk or fermented milk that does not generate curds during the manufacturing process of the milk or fermented milk is obtained by acting an enzyme-containing composition having a peroxidase activity and a protease activity at a certain ratio for a long time, and the present invention has been completed.

That is, the present invention provides the following [1] to [14]:

[1] An enzyme-containing composition having a ratio of the protease activity to the peroxidase activity of 10 or less;

[2] The enzyme-containing composition according to [1], wherein the ratio of the protease activity to the peroxidase activity is 2.5 or less;

[3] The enzyme-containing composition according to [1] or [2], wherein the ratio of the protease activity to the peroxidase activity is 0.01 or more;

[4] The enzyme-containing composition according to [1], containing an enzyme having a peroxidase activity and a protease activity or containing peroxidase and protease, wherein the ratio of the protease activity to the peroxidase activity is 0.01 or more and 10 or less;

[5] The enzyme-containing composition according to [1] or [2], wherein the peroxidase activity is 1 U/mL or more and 100 U/mL or less;

[6] The enzyme-containing composition according to [1] or [2], wherein the protease activity is 0.01 U/mL or more and 250 U/mL or less;

[7] The enzyme-containing composition according to [1] or [2], further comprising a stabilizing agent;

[8] The enzyme-containing composition according to [7], wherein the stabilizing agent is one or more selected from the group consisting of sugar alcohols, monosaccharides, oligosaccharides, and salts;

[9] The enzyme-containing composition according to [1] or [2], wherein the contained enzyme is at least one selected from the group consisting of catalase, peroxidase, catalase-peroxidase, manganese peroxidase, and lactoperoxidase;

[10] The enzyme-containing composition according to [1] or [2], wherein the contained enzyme is catalase-peroxidase;

[11] The enzyme-containing composition according to [10], wherein the catalase-peroxidase is an enzyme derived from an actinomycete;

[12] The enzyme-containing composition according to [10] or [11], wherein the catalase-peroxidase is the following (a) or (b):

(a) catalase-peroxidase composed of the amino acid sequence set forth in SEQ ID NO: 1; and
(b) catalase-peroxidase composed of an amino acid sequence in which from one to several amino acid residues in the amino acid sequence set forth in SEQ ID NO: 1 are deleted, substituted, or inserted and having 80% or more sequence identity with the amino acid sequence set forth in SEQ ID NO: 1;

[13] A method for manufacturing milk, comprising a sterilization step of sterilizing raw milk and a filling step of filling a container with the sterilized raw milk and then sealing the container, wherein
the enzyme-containing composition according to [1] is added to the sterilized raw milk during the period after the sterilization step and before the completion of the filling step; and

[14] A method for manufacturing fermented milk, comprising a heat sterilization step of sterilizing raw milk, an addition step of adding yeast or lactic acid bacteria to the heat-sterilized raw milk, a fermentation step of obtaining fermented milk, and a filling step of filling a container with the obtained fermented milk, wherein
the enzyme-containing composition according to [2] is added to the sterilized raw milk during the period after the heat sterilization step and before the completion of the filling step.

Advantageous Effects of Invention

The present invention facilitates to provide an enzyme-containing composition that is less likely to generate curds by the action of peroxidase even when the peroxidase is reacted with the milk for a long time.

Based on the enzyme-containing composition of the present invention, the invention of a method for manufacturing milk, and the invention of a method for manufacturing fermented milk, it is possible to decrease the amount of hydrogen peroxide included in a dairy product.

The method for manufacturing fermented milk of the present invention facilitates to obtain a lactic acid bacteria proliferation-promoting effect and a fermentation-promoting effect.

Brief Description of Drawings

Fig. 1 illustrates a graph showing the hydrogen peroxide concentrations after making commercially available milk and a mixture of the milk with 10 $\mu$M hydrogen peroxide added react with peroxidase at predetermined concentrations.
Fig. 2 illustrates graphs showing the hydrogen peroxide concentrations after making commercially available milk and a mixture of the milk with 10 $\mu$M hydrogen peroxide added react with peroxidase at predetermined concentrations for predetermined periods of time.

Fig. 3 illustrates graphs showing that an increase in the number of lactic acid bacteria, fermentation-promoting effect, and a decrease in the amount of hydrogen peroxide during the fermentation are observed by using the enzyme-containing composition of the present invention in manufacturing of fermented milk.

Fig. 4 illustrates graphs showing a change in the number of lactic acid bacteria and the number of bifidobacteria during the production of YC380 + BB12 fermented milk by adding the enzyme-containing composition (HP-I) of the present invention.

Fig. 5 illustrates graphs showing a change in pH during the production of YC380 + BB12 fermented milk by adding the enzyme-containing composition (HP-I) of the present invention and a change in the number of bifidobacteria during storage of the fermented milk.

Fig. 6 illustrates tables showing a change in the concentration of hydrogen peroxide during the production of YC380 + BB12 fermented milk by adding the enzyme-containing composition (HP-I) of the present invention and a change in the concentration of hydrogen peroxide during storage of the fermented milk.

Fig. 7 illustrates graphs showing changes in the number of lactic acid bacteria and the number of bifidobacteria during the production of Bifidus fermented milk by adding the enzyme-containing composition (HP-I) of the present invention.

Fig. 8 illustrates graphs showing a change in pH during the production of Bifidus fermented milk by adding the enzyme-containing composition (HP-I) of the present invention and a change in the number of bifidobacteria during storage of the fermented milk.

Fig. 9 illustrates tables showing a change in the concentration of hydrogen peroxide during the production of Bifidus fermented milk by adding the enzyme-containing composition (HP-I) of the present invention and a change in the concentration of hydrogen peroxide during storage of the fermented milk.

Fig. 10 illustrates graphs showing changes in the number of lactic acid bacteria and the number of bifidobacteria during the production of Nature Megumi fermented milk by adding the enzyme-containing composition (HP-I) of the present invention.

Fig. 11 illustrates graphs showing a change in pH during the production of Nature Megumi fermented milk by adding the enzyme-containing composition (HP-I) of the present invention and a change in the number of bifidobacteria during storage of the fermented milk.

Fig. 12 illustrates tables showing a change in the concentration of hydrogen peroxide during the production of Nature Megumi fermented milk by adding the enzyme-containing composition (HP-I) of the present invention and a change in the concentration of hydrogen peroxide during storage of the fermented milk.

Fig. 13 illustrates graphs showing changes in the number of lactic acid bacteria and the number of bifidobacteria during the production of Milmil + YC380 fermented milk by adding the enzyme-containing composition (HP-I) of the present invention.

Fig. 14 illustrates graphs showing a change in pH during the production of Milmil + YC380 fermented milk by adding the enzyme-containing composition (HP-I) of the present invention and a change in the number of bifidobacteria during storage of the fermented milk.

Fig. 15 illustrates tables showing a change in the concentration of hydrogen peroxide during the production of Milmil + YC380 fermented milk by adding the enzyme-containing composition (HP-I) of the present invention and a change in the concentration of hydrogen peroxide during storage of the fermented milk.

Fig. 16 illustrates graphs showing changes in the number of lactic acid bacteria and the number of bifidobacteria during the production of Oishii Yoghurt fermented milk by adding the enzyme-containing composition (HP-I) of the present invention.

Fig. 17 illustrates a graph showing a change in pH during the production of Oishii Yoghurt fermented milk by adding the enzyme-containing composition (HP-I) of the present invention.

Fig. 18 illustrates a table showing a change in the concentration of hydrogen peroxide during the production of Oishii Yoghurt fermented milk by adding the enzyme-containing composition (HP-I) of the present invention.

Fig. 19 illustrates graphs showing changes in the number of lactic acid bacteria and the number of bifidobacteria during the production of Nyu Mild Yoghurt fermented milk by adding the enzyme-containing composition (HP-I) of the present invention.

Fig. 20 illustrates a graph showing a change in pH during the production of Nyu Mild Yoghurt fermented milk by adding the enzyme-containing composition (HP-I) of the present invention.

Fig. 21 illustrates a table showing a change in the concentration of hydrogen peroxide during the production of Nyu Mild Yoghurt fermented milk by adding the enzyme-containing composition (HP-I) of the present invention.

Description of Embodiments

<Enzyme-containing composition>

One aspect of the enzyme-containing composition of the present invention is characterized in that the ratio of the protease activity to the peroxidase activity is 10 or less.

When the ratio of the protease activity to the peroxidase activity is 10 or less, even when the enzyme-containing composition is added to a dairy product, hydrogen peroxide can be reduced without generating unexpected curds.

When the ratio above is greater than 10, there is an increased risk that will lead to unexpected curds in a dairy product. The unexpected curds are increased with increases in storage temperature and number of storage days of the dairy product to which the enzyme-containing composition has been added.

The above ratio is preferably 8 or less, more preferably 5 or less, and more preferably 2.5 or less.

When the enzyme-containing composition is used in manufacturing milk, the ratio of the protease activity to the peroxidase activity is preferably 10 or less. When the enzyme-containing composition is used in manufacturing of fermented milk, the ratio of the protease activity to the peroxidase activity is preferably 2.5 or less.

The lower limit of the above ratio is not particularly limited, but from the viewpoint of preventing generation of unexpected curds and in considering manufacturing of an enzyme-containing composition, high purification leads to poor economic efficiency, and thus, the ratio is preferably 0.01 or more, more preferably 0.05 or more, further more preferably 0.1 or more, and particularly preferably 0.5 or more. This facilitates to increase the number of lactic acid bacteria and to impart a fermentation-promoting effect in the method for manufacturing fermented milk.

A preferred aspect of the present invention is an enzyme-containing composition having a ratio of the protease activity to the peroxidase activity of 0.01 or more and 10 or less. Another preferred aspect of the present invention is an enzyme-containing composition having a ratio of the protease activity to the peroxidase activity of 0.01 or more and 2.5 or less.

The enzyme-containing composition having a ratio of the protease activity to the peroxidase activity of 0.01 or more and 10 or less may involve an aspect of a composition containing an enzyme having a peroxidase activity and a protease activity and an aspect of a composition containing peroxidase and protease.

Another preferred aspect of the present invention is an enzyme-containing composition containing an enzyme having a peroxidase activity and a protease activity or containing peroxidase and protease and having a ratio of the protease activity to the peroxidase activity of 0.01 or more and 10 or less. Another preferred aspect of the present invention is an enzyme-containing composition containing an enzyme having a peroxidase activity and a protease activity or containing peroxidase and protease and having a ratio of the protease activity to the peroxidase activity of 0.01 or more and 2.5 or less.

The peroxidase activity in the enzyme-containing composition of the present invention is preferably 1 U/mL or more, and more preferably 10 U/mL or more. As the peroxidase activity becomes higher, the better hydrogen peroxide removability is achieved. However, since too much concentration may cause problems in e.g. fluidity, the activity is preferably from 1 to 100 U/mL.

The peroxidase activity in the enzyme-containing composition of the present invention means the value measured by the measurement method described later.

The protease activity in the enzyme-containing composition of the present invention is not particularly limited but is preferably 250 U/mL or less. Lower protease activity makes the dairy product less likely to curdle unexpectedly. The protease activity in the enzyme-containing composition of the present invention is preferably 250 U/mL or less, preferably 100 U/mL or less, and more preferably 40 U/mL or less.

From the viewpoint of preventing production of unexpected curds and in considering manufacturing of an enzyme-containing composition, high purification leads to poor economic efficiency, and thus the lower limit of the protease activity in the enzyme-containing composition of the present invention is preferably 0.01 U/mL or more, more preferably 0.05 U/mL or more, further more preferably 0.1 U/mL or more, and particularly preferably 0.5 U/mL or more. This makes it easier to obtain increase in the number of lactic acid bacteria and fermentation-promoting effect.

The protease activity in the enzyme-containing composition of the present invention means the value measured by the measurement method described later.

One aspect of the enzyme-containing composition of the present invention is characterized in that the composition contains an enzyme having a peroxidase activity and a protease activity, or contains peroxidase and protease, wherein the ratio of the peroxidase activity and the protease activity is in the range above. That is, the enzyme contained in the composition of the present invention includes enzymes having a peroxidase activity and a protease activity or two kinds of enzymes of peroxidase and protease. The enzyme to be used in the composition of the present invention refers to a substance that includes one or two kinds of proteins having these enzymatic activities and is added to food or food raw materials mainly for imparting enzymatic activities thereto.

The peroxidase activity in an enzyme having a peroxidase activity and a protease activity or in peroxidase and protease can be verified by a peroxidase activity measurement method described later. The protease activity can be verified by a protease activity measurement method described later.

Examples of the enzyme that can be used in the present invention include catalase, peroxidase, catalase-peroxidase, manganese peroxidase, and lactoperoxidase. These enzymes can be used alone or in combination of two or more. Among them, catalase-peroxidase is preferably used.

As the protease, as long as a protease activity can be observed by the protease activity measurement method described later, a commercially available enzyme can be used.

The origin of the enzyme that can be used in the present invention is not limited and may be derived from an animal, plant, or microorganism. It is preferable to use an enzyme having a peroxidase activity derived from a microorganism because of its easiness to use in manufacturing.

Use of the peroxidase activity measurement method described later facilitates to search the natural world for microorganisms and so on having a peroxidase activity.

Examples of the microorganism producing an enzyme having a peroxidase activity are filamentous fungi, actinomycetes, and bacteria.

Among these enzymes, it is preferable to use catalase-peroxidase derived from an actinomycete.

As the catalase-peroxidase, it is preferable to use (a) catalase-peroxidase composed of the amino acid sequence set forth in SEQ ID NO: 1 or (b) catalase-peroxidase composed of an amino acid sequence in which from one to several amino acid residues in the amino acid sequence set forth in SEQ ID NO: 1 are deleted, substituted, or inserted and having 80% or more sequence identity with the amino acid sequence set forth in SEQ ID NO: 1.

SEQ ID NO: 2 shows the gene sequence of the amino acid sequence of SEQ ID NO: 1.

The amino acid sequence set forth in SEQ ID NO: 1 is catalase-peroxidase produced by Streptomyces achromogenes. The catalase-peroxidase may be catalase-peroxidase that is not derived from Streptomyces achromogenes as long as the amino acid sequence is the same as or has 80% or more sequence identity with the catalase-peroxidase derived from Streptomyces achromogenes. The catalase-peroxidase may be not only a polypeptide but also a glycopeptide as long as its amino acid sequence is the same as or has 80% or more sequence identity with the catalase-peroxidase derived from Streptomyces achromogenes.

In catalase-peroxidase in which from one to several amino acid sequences in the amino acid sequence set forth in SEQ ID NO: 1 are deleted, substituted, or inserted, the number of the deleted, substituted, or inserted amino acid residues is not limited as long as the catalase-peroxidase has an enzyme activity equivalent to that of catalase-peroxidase composed of the amino acid sequence set forth in SEQ ID NO: 1, but the number is preferably from 1 to 20, further preferably from 1 to 10, and further preferably from 1 to 8.

The sequence identity between the catalase-peroxidase with deletion, substitution, or insertion and the amino acid sequence of SEQ ID NO: 1 is preferably 80% or more, more preferably 85% or more, further more preferably 90% or more, further more preferably 95% or more, and further more preferably 99% or more. Such an identity percentage of a sequence can be calculated using published or commercially available software having algorithm for comparing the sequence with a reference sequence as an inquiry sequence. As an example, BLAST, FASTA, or GENETYX (manufactured by Software Development Co., Ltd.) can be used.

When a microorganism or the like having a high peroxidase activity is found from the natural world, a method of further enhancing the peroxidase activity by breeding the microorganism or the like and a method for production using another microorganism or the like are exemplified.

As the method for breeding the microorganism or the like, a method for promoting mutation in the microorganism or the like by using ultraviolet rays, radiation, a chemical substance, or the like can be used.

Examples of the method of producing protein having a peroxidase activity by using another microorganism or the like include a method by purifying and identifying the protein, then obtaining gene information of the peroxidase, amplifying to obtain the gene by PCR or the like, and then introducing the gene into a host microorganism. The protein having a peroxidase activity can be obtained by cultivating the host microorganism into which the gene has been introduced. Any vector may be used to introduced into the host microorganism. Any microorganism may be used as the host microorganism.

The host microorganism is not particularly limited as long as it can produce protein having a peroxidase activity, but, for example, genus Bacillus, genus Paenibacillus, genus Brevibacillus (e.g., Brevibacillus chosinensis), genus Escherichia (e.g., Escherichia coli), genus Corynebacterium, genus Saccharomyces, genus Shizosaccharomyces, genus Kluyveromyces, genus Pichia, genus Aspergillus, genus Penicillium, genus Trichoderma, and microorganisms, which have been eaten, such as lactic acid bacteria and acetic acid bacteria, may be used as the target.

The form of the enzyme-containing composition of the present invention may be a solid, or may be a liquid. Whether the enzyme-containing composition of the present invention is a solid or a liquid can be determined as appropriate in accordance with the purpose of use.

When the subject to which the enzyme is added is a solid, the enzyme-containing composition is preferably used in a solid form, because the subject is unlikely to be diluted by the enzyme-containing composition. Even if the enzyme-containing composition is in a liquid form, the composition can be preferably used by increasing the enzymatic activity even if the subject to be added is a solid.

When the subject to which the enzyme is added is a liquid, the enzyme-containing composition is preferably used in a liquid form. The workability when the enzyme-containing composition is added to a subject is enhanced.

The enzyme-containing composition of the present invention preferably contains a stabilizing agent.

Examples of the stabilizing agent that is used in the enzyme-containing composition of the present invention include sugar alcohols such as glycerin and sorbitol; monosaccharides or oligosaccharides such as glucose, sucrose, and trehalose; and salts such as sodium chloride.

When a stabilizing agent is contained in the solid enzyme-containing composition, it is preferable to use a stabilizing agent that is a solid at room temperature (20°C). As such a stabilizing agent, for example, a sugar alcohol such as sorbitol; a monosaccharide or oligosaccharide such as glucose, sucrose, or trehalose; or a salt such as sodium chloride can be used.

When a stabilizing agent is contained in the liquid enzyme-containing composition, it is preferable to use a stabilizing agent that is a liquid at room temperature (20°C) or a solid stabilizing agent that has excellent solubility in water. As such a stabilizing agent, a sugar alcohol such as glycerin or sorbitol can be used.

The amount of the stabilizing agent contained in the enzyme-containing composition of the present invention is preferably from 10 to 90 mass%, more preferably from 20 to 80 mass%, and further more preferably from 25 to 75 mass%. The stabilizing agent contained in this range maintains the storage stability of the peroxidase and is also economically advantageous.

The pH of the enzyme-containing composition of the present invention is, from the viewpoint of maintaining the peroxidase for a long time, preferably from 5.0 to 9.0, more preferably from 5.5 to 8.5, and further more preferably from 6.0 to 8.0. The pH of a liquid enzyme-containing composition may be measured as it is. In a solid enzyme-containing composition, the pH can be measured by dissolving the composition in water.

The storage temperature of the enzyme-containing composition of the present invention is, from the viewpoint of preventing a reduction in the peroxidase activity, preferably as low as possible, more preferably 30°C or less, and more preferably 10°C or less.

The enzyme-containing composition of the present invention may contain various ingredients as needed. Examples thereof include a metal salt that contributes to stabilization of peroxidase, various types of sugars, ascorbic acid, starch as an excipient to improve usability, dextrin, and an inorganic salt having a buffering action.

<Method for manufacturing milk>

Among the present invention, the method for manufacturing milk encompasses a sterilization step of sterilizing raw milk and a filling step of filling a container with the sterilized raw milk and then sealing the container, wherein the enzyme-containing composition is added to the sterilized raw material during the period after the sterilization step and before the completion of the filling step. Here, the ratio of the protease activity to the peroxidase activity of the enzyme-containing composition that is used in the method for manufacturing milk is preferably 10 or less.

In the present invention, the temperature conditions in the sterilization step are heat sterilization at from 120°C to 150°C for from 1 to 3 seconds or heat sterilization by a method having bactericidal effect equal to or higher than the above. When the temperature is lower than the above conditions, since at least one selected from the group consisting of lactoperoxidase and catalase contained in raw milk partially remains in the active form, the hydrogen peroxide in the milk can be removed by the lactoperoxidase or catalase, even if the enzyme-containing composition of the present invention is used, almost no effect is obtained.

In the present invention, sterilized raw milk refers to milk having substantially no lactoperoxidase activity and catalase activity, specifically, sterilized raw milk of which the hydrogen peroxidase concentration does not decrease when hydrogen peroxide is added to the milk and then the milk is left to stand.

The period after the sterilization step and before the completion of the filling step means a period allowing the peroxidase derived from the enzyme-containing composition to be present in the active form in the filled and packaged milk. For example, even if the enzyme-containing composition is added to milk immediately after the sterilization step, the peroxidase included in the enzyme-containing composition is deactivated by the heat of the sterilized milk, and is therefore such addition is unpreferable. The composition may be added to the sterilized milk that has been cooled to an extent that all or part of the peroxidase activity is not lost after the sterilization step.

The period before the completion of the filling step may be any period as long as it is before the milk is sealed after filling. The addition of the enzyme-containing composition may be performed at the same time as the filling step. Alternatively, the enzyme-containing composition may be added to a container filled with the milk, and then the container may be sealed.

That is, after the lactoperoxidase activity and catalase activity included in raw milk are inactivated in the sterilization step, and before the sterilized milk is filled into the container and the container is sealed, the enzyme-containing composition of the present invention may be added to the sterilized raw milk.

<Method for manufacturing fermented milk>

Among the present invention, the method for manufacturing fermented milk encompasses a heat sterilization step of heat-sterilizing raw milk, an addition step of adding yeast or lactic acid bacteria to the raw milk after the heat sterilization, a fermentation step of obtaining fermented milk, and a filling step of filling a container with the obtained fermented milk, wherein the enzyme-containing composition is added to the raw material after the heat sterilization during the period after the heat sterilization step and before the completion of the filling step. Here, the ratio of the protease activity to the peroxidase activity of the enzyme-containing composition that is used in the method for manufacturing milk is preferably 2.5 or less.

In the method for manufacturing fermented milk of the present invention, the temperature conditions in the heat sterilization step may be for performing sterilization at from 70°C to 110°C for from 30 seconds to 10 minutes, and the method may be a method having bactericidal effect that is equal to or higher than the above.

The heating temperature is preferably from 75°C to 105°C, more preferably from 80°C to 100°C, and further preferably from 85°C to 95°C.

The heating time is preferably from 1 to 10 minutes, more preferably from 2 to 8 minutes, and further more preferably from 3 to 7 minutes.

The heating temperature and the heating time in the heat sterilization step can be used in combination.

Yeast or lactic acid bacteria are added to heat-sterilized raw milk that has been cooled to a temperature (from 25°C to 43°C) suitable for fermentation of the yeast or lactic acid bacteria.

The fermentation temperature of yeast is preferably from 25°C to 40° and more preferably from 30°C to 37°C.

The fermentation temperature of lactic acid bacteria is preferably from 30°C to 43°C and further preferably from 37°C to 43°C.

The number of the yeast or lactic acid bacteria that are added to the heat-sterilized raw milk can be appropriately adjusted. The amount of the yeast or lactic acid bacteria that are added to the heat-sterilized raw milk is preferably from $1 \times 10^4$ to $1 \times 10^{10}$ cells/mL from the viewpoint of maintaining the fermentation speed and preventing the increase in manufacturing cost.

When yeast is used in the method for manufacturing fermented milk of the present invention, the type of the yeast is not particularly limited. As the yeast, for example, lactose-fermenting yeast or lactose-nonfermenting yeast is used. When lactose-nonfermenting yeast is used, it is preferable to add a fermentable sugar source, such as sucrose or glucose, to raw milk in an amount of from 0.1% to 10% in advance.

Examples of the lactic acid bacteria that are used in the method for manufacturing fermented milk of the present invention include microorganisms belonging to genus Lactobacillus or genus Streptococcus.

Specifically, the examples include Lactococcus lactis, Lactococcus lactis subsp. cremoris, Lactobacillus casei, Lactobacillus gasseri, Lactobacillus rhamnosus, Lactobacillus acidophilus, Lactobacillus plantarum, Lactobacillus brevis, Lactobacillus delbrueckii subsp. bulgaricus, and Streptococcus thermophilus. Bifidobacteria belonging to genus Bifidobacterium may be used as the lactic acid bacteria. These lactic acid bacteria (including bifidobacteria) may be used alone or in combination of two or more.

The fermented milk obtained by using Lactobacillus delbrueckii subsp. bulgaricus and Streptococcus thermophilus, among the above-mentioned lactic acid bacteria, is yoghurt in the narrow sense.

The period after the heat sterilization step and before the completion of the filling step means a period allowing the peroxidase derived from the enzyme-containing composition to be present in the active form in the filled and packaged milk.

The period before the completion of the filling step does not limit the timing for adding the enzyme-containing composition as long as it is before the milk is sealed after filling. For example, the enzyme-containing composition may be added to the heat-sterilized raw milk after it is cooled. Alternatively, the enzyme-containing composition may be added to the heat-sterilized raw milk after cooling at approximately the same time as yeast or lactic acid bacteria are added.

In the present invention, approximately the same indicates a relative one and what changes in accordance with the growth rate of the yeast or lactic acid bacteria used and means that the yeast or lactic acid bacteria used correspond to the induction period. The enzyme-containing composition is preferably added to the yeast or lactic acid bacteria to be used during the induction period in order to prevent the production of unexpected curds.

In the raw milk to which the enzyme-containing composition is added, the pH decreases with the progress of fermentation. The pH of the raw milk after the completion of fermentation (fermented milk) is preferably from 3.5 to 5.5, more preferably from 4.0 to 5.0, and further more preferably from 4.2 to 4.7.

It is preferable that the peroxidase activity in the enzyme-containing composition added to raw milk decrease with the progress of fermentation of the raw milk. Examples

The present invention will now be described with reference to Examples but the present invention is not limited to the Examples.

<Evaluation method>

(Method for measuring peroxidase activity)

A potassium phosphate buffer (100 mmol/L) was prepared by dissolving potassium dihydrogen phosphate (13.61 g) in water, adjusting the pH to 6.5 with a sodium hydroxide reagent (1 mol/L), and adding water thereto up to 1 L.

A hydrochloric acid reagent (0.1 mol/L) was prepared by adding water to 1 mol/L hydrochloric acid (100 mL) up to 1,000 mL.

A hydrogen peroxide reagent (100 mmol/L) was prepared by adding water to a 30% hydrogen peroxide solution (1.1 g) up to 100 mL. To the hydrogen peroxide reagent (100 mmol/L, 1 mL) was added water up to 4 mL to give a hydrogen peroxide reagent (25 mmol/L), and the absorbance thereof at a wavelength of 240 nm was measured, and the Factor was calculated by $100 \times$ (measured value)/[theoretical value (1.09)]. Subsequently, a 100 mmol/L hydrogen peroxide reagent was precisely prepared by weighing 100/Factor $\times$ 1.1 g of a hydrogen peroxide solution (30%) and adding water thereto up to 100 mL.

A pyrogallol reagent (396.5 mmol/L) was prepared by dissolving pyrogallol (0.5 g) in water up to 10 mL.

In a test tube (thickness: 0.8 mm), a potassium phosphate buffer (405 μL), the pyrogallol reagent (396.5 mmol/L, 270 μL), the hydrogen peroxide reagent (100 mmol/L, 135 μL), and water (1.79 mL) were mixed and were preincubated at 30°C for 10 minutes.

The above mixture was mixed with an appropriately diluted enzyme-containing composition (liquid enzyme-containing composition, 100 μL), followed by incubation at 30°C for 10 minutes.

As a Blank, MQ (100 μL) was used instead of the enzyme.

At 10 minutes' time after the reaction, the hydrochloric acid reagent (0.1 mol/L, 300 μL) was added to and mixed with the solution above to stop the enzyme reaction, and the absorbance at a wavelength of 420 nm was measured.

An enzyme amount that generates purpurogallin (1 mg) at pH 6.5 at 30°C for 20 seconds was defined as one unit.

U/mL = ΔOD (OD test - OD blank)/12 $\times$ 3/0.1 $\times$ (dilution degree) $\times$ 20/600

(Measurement method of protease activity)

As the dilution buffer for the enzyme-containing composition, a 50 mM tris-hydrochloric acid buffer (pH 7.5) containing 2 mM calcium acetate was used.

A 0.1 M tris solution was prepared by dissolving tris(hydroxymethyl)aminomethane (12.11 g) in purified water up to 1 L.

A 0.2 M calcium acetate solution was prepared by dissolving calcium acetate monohydrate (MW: 176.18, 3.52 g) in purified water up to 100 mL.

A precipitation reagent was prepared by dissolving trichloroacetic acid (18.0 g) and anhydrous sodium acetate (18.0 g) in distilled water (700 mL), adding acetic acid (19.8 g, 18.9 mL) thereto, verifying that the pH to be 4 $\pm$ 0.05 (when the pH is out of the range, the pH was adjusted with 1 N NaOH or HCl), and then adding purified water up to 1 L.

Substrate solution (preparaed at time of use): milk casein (1.2 g, manufactured by CALBIOCHEM, Production No. 218682) was precisely weighed and was added little by little with shaking to purified water (about 20 mL) stirred up to make a suspension, 0.1 M tris solution (100 mL) was added to the suspension, the resulting mixture was sufficiently stirred, then heated at 60°C for 10 minutes, and cooled by a water bath, calcium acetate solution (2 mL) was added thereto, the pH was adjusted with 1 N hydrochloric acid to 7.5, and purified water was added thereto up to 200 mL.

In a test tube, a sample solution (liquid enzyme-containing composition, 0.5 mL) was put. The solution was incubated at 30°C for 3 to 5 minutes, and the substrate solution (2.5 mL) was added to and mixed with the solution, followed by reaction at 30°C for 10 minutes. Subsequently, the precipitation reagent (2.5 mL) was added to and mixed with the reaction solution, followed by leaving to stand at 30°C for 30 minutes. Then, filtration through filtration paper No. 4A was performed (30 minutes or more), and the absorbance of the filtrate at 275 nm was measured. In a blank, the order of putting the substrate solution and the precipitation reagent was reversed.

$$U/mL = \Delta OD \times F \times dilution\ degree,$$

where F is a conversion factor determined from a tyrosine calibration curve.

(Measurement method of hydrogen peroxide concentration)

One hundred microliters of 20% sulfosalicylic acid was mixed with 900 μL of a milk sample (milk or fermented milk (appropriately mixed)), and 180 μL of a coloring reagent was added to 20 μL of the centrifugal (14,800 rpm, 4°C) supernatant of the mixture. The resulting mixture was left to stand at room temperature for 30 minutes, and the absorbance at 595 nm was then measured. Separately, a calibration curve was formed using hydrogen peroxide solutions with known concentrations, and the hydrogen peroxide concentration in the sample was calculated based on the calibration curve.

Coloring reagent: mixture of R1* and R2** at 1 : 100,
R1 (25 mM ammonium iron(II) sulfate hexahydrate, 2.5 M sulfuric acid); and
R2 (100 mM sorbitol, 125 μM xylenol orange).

[Reference Example 1: Search for microorganism]

A microorganism having a peroxidase activity was searched using microbial strains possessed by Godo Shusei Co., Ltd., and a peroxidase activity was observed in the culture of an actinomycete. The culture was purified to obtain the protein of SEQ ID NO: 1 (hereinafter, may be referred to as HP-I), a peroxidase activity thereof was confirmed, and the gene sequence of SEQ ID NO: 2 of catalase-peroxidase HPI (WP_030611371.1) in Streptomyces achromogenes subsp. achromogenes (Strain: NRRL B-2120, genome information included) was obtained.

[Example 1: Production by commercially available strain]

The above NRRL B-2120 strain was cultured to obtain a culture broth reached the stationary phase. That is, a culture medium (80 mL, 1% glucose, 1% yeast extract KAT, 0.2% ammonium dihydrogen phosphate, and 0.1% magnesium sulfate) was prepared in a 500-mL flask and cultured at 210 rpm at 30°C. Four milliliters of the culture broth was inoculated in the culture medium and cultured under the same conditions for 72 hours. The culture solution was centrifuged at 15,000 rpm for 10 minutes at 4°C, and the centrifuged precipitate was suspended in a 0.1 M phosphate buffer (pH 6.5) and then ultrasonicated for 15 minutes. The sonicated solution was centrifuged at 15,000 rpm for 10 minutes at 4°C, and the resulting centrifugal supernatant (9.5 mL) was dialyzed with a 20 mM phosphate buffer overnight (fraction molecular weight: from 12,000 to 14,000). Ten milliliters of the dialyzed sample was applied to a DEAE-toyopearl column ($\phi$15 × 110 mm, volume: about 20 mL) equilibrated by the buffer above and was eluted with a step-wise gradient of NaCl (0.1, 0.15, and 0.2 M) under conditions of a flow rate of 1 mL/min. The 0.2 M fraction was collected and was dialyzed with a 20 mM phosphate buffer (pH 6.5) again. One milliliter of the dialyzed sample was applied to NGC Enrich-Q (negative ion exchange column) to perform purification at a flow rate of 1 mL/min with a linear gradient of NaCl from 0 to 0.8 M to obtain a liquid enzyme-containing composition.
The peroxidase activity of the enzyme-containing composition was 0.01 U/mL, and no protease activity was detected.

[Example 2: Production by host]

As the primers for amplifying the gene sequence of SEQ ID NO: 2, the following primers 1 and 2 were used:

Primer 1: 5'-TAGAATTCCATATGACTGAGAACCACGAC-3' (SEQ ID NO: 3); and
Primer 2: 5'-CCCCAAGCTTTCAATGATGATGATGATGATGGACGAGGTCGAAGCG-3' (SEQ ID NO: 4).

The amplified gene was ligated into the PET28a vector, and the resulting plasmid was introduced into Escherichia coli BL21 stain. Expression of the transformant was induced in an LB medium to perform culture. The bacterial cells were collected by centrifugation, and the bacterial cell extract obtained by ultrasonication was purified by an Ni column to obtain a liquid enzyme-containing composition.
The peroxidase activity of the enzyme-containing composition was 2.0 U/mL, and no protease activity was detected.

[Example 3]

12-5 strain selected by irradiating NRRL B-2120 strain with ultraviolet rays was inoculated in 100 mL of a soybean flour medium (in which 1% glycerin, 1% soybean flour, 0.5% yeast extract, 0.5% casamino acid, 0.4% calcium carbonate, and a metal salt solution 0.1% (the metal salt solution was an aqueous solution of 0.1% iron sulfate heptahydrate, 0.09% zinc sulfate, and 0.02% manganese sulfate) were sterilized and then separately sterilized glucose was added thereto in an amount of 1%) in a 500-mL flask and cultured at 210 rpm at 30°C for from 2 to 3 days. The culture solution was centrifuged at 15,000 rpm for 10 minutes at 4°C to collect cells, and the cells were stored frozen until use.

The frozen cells were thawed in a potassium phosphate buffer (pH 6.5) so as to give 0.15 g/mL cells and 0.21% egg-white lysozyme, and the resulting solution was stirred at 37°C for 5 hours to extract enzymes.

In this stage, the peroxidase activity was 1.2 U/mL, the protease activity was 138 U/mL, and the ratio of the protease activity to the peroxidase activity was 115. This may be referred to as the enzyme-containing composition before purification.

This lysozyme extraction solution, 9.6% $K_2HPO_4$ solution, and 11.52% $CaCl_2 \cdot 2H_2O$ solution were mixed at a ratio of 100 : 10 : 10 such that the final concentrations of $K_2HPO_4$ and $CaCl_2 \cdot 2H_2O$ were 0.8% and 0.96%, respectively, to generate calcium phosphate gel. Silica 300S was mixed with the gel at 1%, and the mixture was filtered through filter paper coated with about 1.5 mm Silica 300S. Activated carbon PL-CPS2 (manufactured by Dainen Co., Ltd.) mixed with this filtrate at 2%, followed by stirring at room temperature for 2 hours to absorb and remove protease. Silica 100F was mixed with the filtrate at 1%, and the mixture was filtered through filter paper coated with about 1.5 mm Silica 100F. The filtrate was concentrated by ultrafiltration or the like to give an arbitrary activity, and 25% (w/w) glycerin was then added thereto as a stabilizing agent to obtain a liquid enzyme-containing composition.

In this enzyme-containing composition, the peroxidase activity was 42 U/mL, the protease activity was 53 U/mL, and the ratio of the protease activity to the peroxidase activity was 1.3. This may be referred to as the enzyme-containing composition after purification.

An enzyme-containing composition having an arbitrary ratio of the protease activity to the peroxidase activity can be prepared using the enzyme-containing composition of Example 2 and the enzyme-containing compositions before and after purification of Example 3.

<Evaluation: Decomposition of hydrogen peroxide in milk>

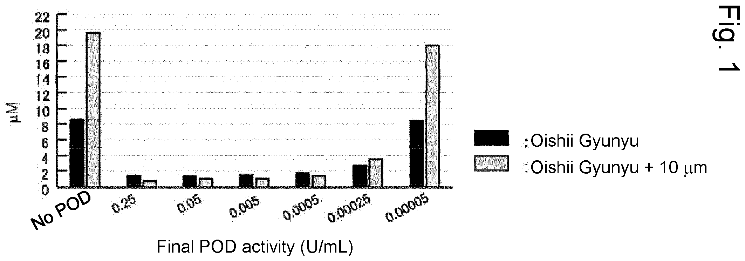

Peroxidase (enzyme-containing composition before purification) of Example 3 was mixed with commercially available milk (trade name: Meiji Oishii Gyunyu) and the milk containing 10 μM hydrogen peroxide at final concentrations of 0.00005, 0.00025, 0.0005, 0.005, 0.05, and 0.25 U/mL, respectively, and the mixtures were left to stand at 4°C for 90 minutes. As a result, the hydrogen peroxide concentration at an activity of 0.05 U/mL or more was about 1 μM (near the limit of determination) (Fig. 1).

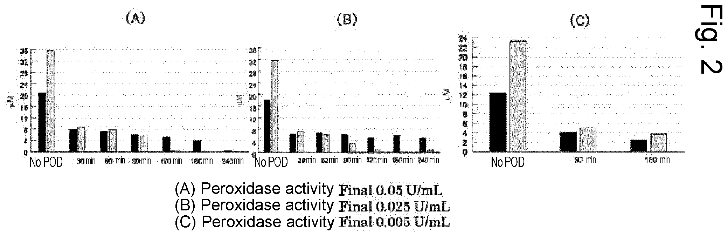

Peroxidase (enzyme-containing composition before purification) of Example 3 was mixed with commercially available milk (trade name: Meiji Oishii Gyunyu) and the milk containing 10 μM hydrogen peroxide at final concentrations of 0.05, 0.025, and 0.005 U/mL, respectively, and the mixtures were left to stand at 4°C for 30, 60, 90, 120, and 240 minutes. As a result, the hydrogen peroxide could be almost completely decomposed by the activity at a final concentration of 0.05 U/mL (less than the limit of determination, the left in Fig. 2 is 0.05 U/mL, the center in Fig. 2 is 0.025 U/mL, and the right in Fig. 2 is 0.005 U/mL).

<Protease concentration that does not affect milk>

Skimmed milk was mixed with commercially available pasteurized milk (trade name: Takanashi Teion Sakkin Gyunyu (Takanashi milk sterilized at low temperature)) such that the skimmed milk concentration was 2%, and the mixture was mixed with the enzyme-containing composition before purification of Example 3 and the enzyme-containing composition of Example 2 such that the final concentrations of the protease activity were adjusted to 2.45, 0.52, 0.25, and 0.049 U/mL, while the peroxidase activity was fixed to a final concentration of 0.04 U/mL (the ratios of the protease activity to the peroxidase activity were 61.3, 13, 6.3, and 1.2 in order). These enzyme-containing compositions were left to stand at 43°C for 7 hours while observing at every 1 hour. The curds were observed within 2 hours at 2.45 U/mL and within from 5 to 6 hours at 0.52 U/mL, but no curds were observed at 0.25 U/mL and 0.049 U/mL. Subsequently, milk coagulation was similarly investigated at protease activities of 0.25, 0.35, 0.45, and 0.52 U/mL (the ratios of the protease activity to the peroxidase activity were 6.3, 8.8, 11.3, and 13 in order). As a result, curds were observed within 5 hours at 0.52 U/mL and within 7 hours at 0.45 U/mL, but no curds were observed at other protease activities. Furthermore, curds were investigated at protease activities of 0.35, 0.4, and 0.45 U/mL (the ratios of the protease activity to the peroxidase activity were 8.8, 10, and 11.3 in order). As a result, at all protease activities, no milk coagulation was observed. That is, it was found that unexpected curds were not observed as long as the ratio of the protease activity to the peroxidase activity was 10 or less.

<Protease concentration that does not affect fermented milk>

Skimmed milk was mixed with commercially available pasteurized milk (trade name: Takanashi Teion Sakkin Gyunyu (Takanashi milk sterilized at low temperature)) such that the skimmed milk concentration was 2%, and the mixture was sterilized by placing in boiling water for 20 minutes. After the sterilization, the milk was cooled to 43°C, and a starter (YC-380, manufactured by Chr. Hansen) brought to room temperature was added thereto at 10 mg/100 g, followed by thoroughly stirring. Subsequently, the resulting yoghurt undiluted solution was mixed with the enzyme-containing composition before purification of Example 3 and the enzyme-containing composition of Example 2 such that the final concentrations of the protease activity were adjusted to 0.5, 0.4, 0.3, 0.2, 0.1, and 0.05 U/mL, while the peroxidase activity was fixed to a final concentration of 0.04 U/mL (the ratios of the protease activity to the peroxidase activity were 12.5, 10, 7.5, 5, and 2.5 in order). These enzyme-containing compositions were left to stand at 43°C for from 0 to 5 hours to investigate the maximum protease activity that did not generate curds before a control caused the generation of curds by fermentation. The control was milk which did not contain the enzyme. As a result, curds were generated within 2 hours at 0.5 U/mL and 0.4 U/mL and within 3 hours at 0.3 U/mL and 0.2 U/mL. These milk samples curdled earlier than the control to which the enzyme was not added. In 0.1 U/mL and 0.05 U/mL, generation of curds due to the influence of protease was not visually observed.

That is, it was found that unexpected curds were not observed as long as the ratio of the protease activity to the peroxidase activity was 2.5 or less.

<Evaluation: Addition to fermented milk 1>

Eight grams of skimmed milk was mixed with 392 g of commercially available pasteurized milk (trade name: Teion Sakkin Gyunyu (manufactured by Takanashi Milk Products Co., Ltd.)) such that the skimmed milk concentration was 2%, and the mixture was sterilized by placing in boiling water for 20 minutes. After the sterilization, the milk was cooled to 43°C, and a starter (YC-380, manufactured by Chr. Hansen) was added thereto at 10 mg/100 g, followed by thoroughly stirring. Into each of two sterilized medium bottles, 99.9 g of the resulting yoghurt undiluted solution was dispensed. An enzyme-containing composition (the ratio of the protease activity to the peroxidase activity was 1.3) was added to one of the bottles such that the final peroxidase activity concentration was 0.04 U/g, the same amount of a 0.1 M potassium phosphate buffer (pH 6.5) was added to the other bottle as a control, and the mixtures were each thoroughly mixed. These mixtures were each dispensed into seven 15-mL tubes in 13 mL portions for fermentation for from 0 to 4 hours, and fermentation was started at 43°C. The fermented solution was sampled from each tube over time, stepwise diluted, then seeded in an appropriate amount on a BCP plate (Eiken Chemical Co., Ltd.), and cultured by leaving to stand at 37°C for 48 hours. After the culturing, the number of cells was counted. Subsequently, the hydrogen peroxide concentration and pH of the yoghurt remained in each of the 15-mL tubes were measured.

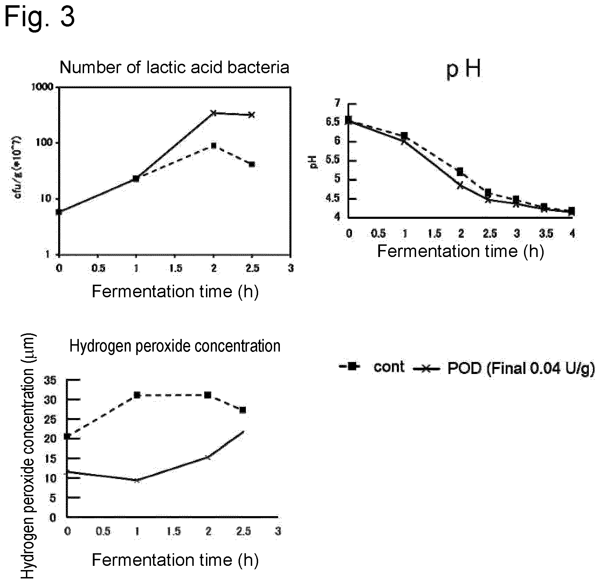

Fig. 3 shows the results. Addition of the enzyme-containing composition increased the number of lactic acid bacteria by 3.8 times in fermentation for 2 hours. The pH also rapidly decreased during fermentation, fermentation-promoting effect of from 20 to 30 minutes was obtained. It was confirmed that the hydrogen peroxide content decreased during fermentation and that generation of curds did not occur by the action of the enzyme-containing composition.

<Evaluation: Addition to fermented milk 2>

Skimmed milk was mixed with commercially available pasteurized milk (Trade name: Teion Sakkin Gyunyu (manufactured by Takanashi Milk Products Co., Ltd.)) such that the skimmed milk concentration was 2%, and the mixture was sterilized by placing in boiling water for 20 minutes. After the sterilization, the milk was cooled to 43°C, and a starter (YC-380, manufactured by Chr. Hansen) and bifidobacteria (BB12, manufactured by Chr. Hansen) were added thereto at 10 mg/100 g and 5 mg/100 g, respectively, followed by thoroughly stirring.

Into each of two sterilized medium bottles, 99.9 g of the resulting yoghurt undiluted solution was dispensed. The enzyme-containing composition after purification of Example 3 (the ratio of the protease activity to the peroxidase activity was 1.3) was added to one of the bottles such that the final peroxidase activity concentration was 0.04 U/g, the same amount of sterilized MilliQ was added to the other bottle as a control, and the mixtures were each thoroughly mixed. These mixtures were each dispensed into 15-mL tubes in 13 mL portions for fermentation for from 0 to 4 hours and a preservation test, and fermentation was started at 43°C. The fermented solution was sampled from each tube over time, stepwise diluted, then seeded in an appropriate amount on a BCP plate (Eiken Chemical Co., Ltd.) for counting the number of lactic acid bacteria and on a TOS plate (Yakult Pharmaceutical Industry Co., Ltd.) for counting the number of bifidobacterial, and cultured by leaving to stand at 37°C for 72 hours. After the culturing, the numbers of cells were counted. Furthermore, after the completion of fermentation, the fermented milk in the 15-mL tubes was stored at 10°C, sampled from the tubes chronologically, stepwise diluted, then seeded in an appropriate amount on a BCP plate (Eiken Chemical Co., Ltd.) for counting the number of lactic acid bacteria and on a TOS plate (Yakult Pharmaceutical Industry Co., Ltd.) for counting the number of bifidobacterial, and cultured by leaving to stand at 37°C for 72 hours. After the culturing, the numbers of cells were counted.

The hydrogen peroxide concentration and pH of the fermented milk remained in the 15-mL tubes after the use in each test were measured.

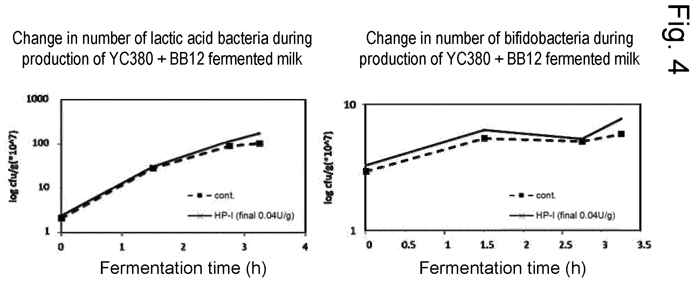

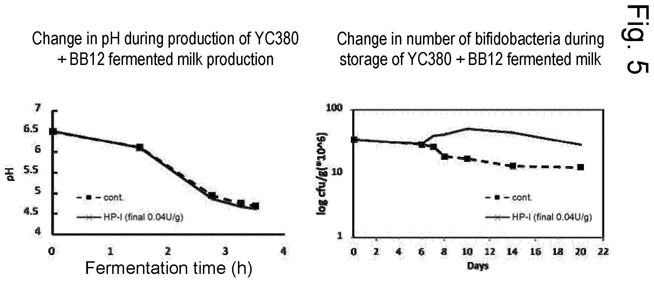

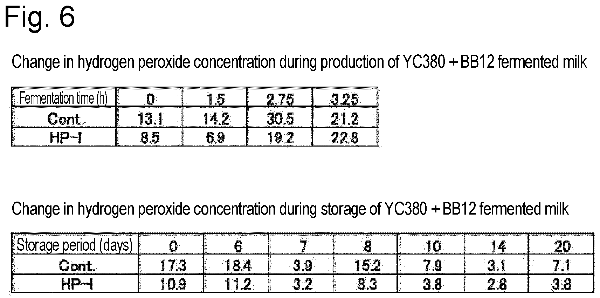

Figs. 4 to 6 show the results. The addition of the enzyme-containing composition (HP-I) after purification of Example 3 increased the number of lactic acid bacteria 1.7 times by fermentation for 3.25 hours (Fig. 4). The addition of the enzyme-containing composition increased the number of bifidobacteria 1.3 times by fermentation for 3.25 hours (Fig. 4). The pH also rapidly decreased during fermentation, and fermentation-promoting effect of about several minutes was obtained (Fig. 5). The addition of the enzyme-containing composition suppressed the decrease rate of the number of bifidobacteria during storage of the fermented milk (Fig. 5). It was confirmed that the hydrogen peroxide content decreased during fermentation and that generation of curds did not occur by the action of the enzyme-containing composition (Fig. 6) .

<Fermented milk produced using commercially available fermented milk> (Preservation test carried out using Bifidus, Nature Megumi, and Milmil)

Skimmed milk was mixed with commercially available pasteurized milk (Trade name: Teion Sakkin Gyunyu (manufactured by Takanashi Milk Products Co., Ltd.)) such that the skimmed milk concentration was 2%, and the mixture was sterilized by placing in boiling water for 20 minutes. After the sterilization, the milk was cooled to 43°C, and the commercially available fermented milk shown in Table 1 was added thereto in the weights shown in the table, followed by sufficiently stirring. Into each of two sterilized medium bottles, 99.9 g of the resulting yoghurt undiluted solution was dispensed. The enzyme-containing composition after purification of Example 3 (the ratio of the protease activity to the peroxidase activity was 1.3) was added to one of the bottles such that the final peroxidase activity concentration was 0.04 U/g, the same amount of sterilized MilliQ was added to the other bottle as a control, and the mixtures were each thoroughly mixed.

These mixtures were each dispensed into 15-mL tubes in 13 mL portions for fermentation for from 0 to 4 hours and a preservation test, and fermentation was started at 43°C. The fermented solution was sampled from each tube over time, stepwise diluted, then seeded in an appropriate amount on a BCP plate (Eiken Chemical Co., Ltd.) for counting the number of lactic acid bacteria and on a TOS plate (Yakult Pharmaceutical Industry Co., Ltd.) for counting the number of bifidobacterial, and cultured by leaving to stand at 37°C for 72 hours. After the culturing, the numbers of cells were counted. Furthermore, after the completion of fermentation, the fermented milk in the 15-mL tubes was stored at 10°C, sampled from the tubes chronologically, stepwise diluted, then seeded in an appropriate amount on a BCP plate (Eiken Chemical Co., Ltd.) for counting the number of lactic acid bacteria and on a TOS plate (Yakult Pharmaceutical Industry Co., Ltd.) for counting the number of bifidobacterial, and cultured by leaving to stand at 37°C for 72 hours. After the culturing, the numbers of cells were counted. The hydrogen peroxide concentration and pH of the fermented milk remained in the 15-mL tubes after the use in each test were measured.

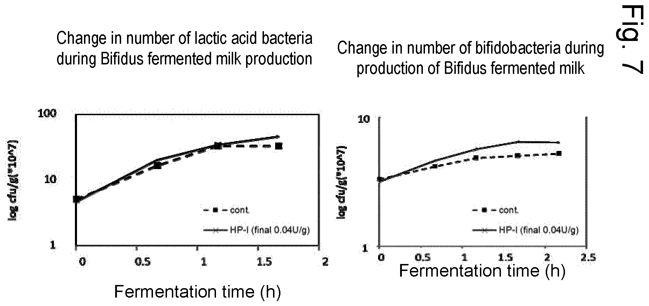

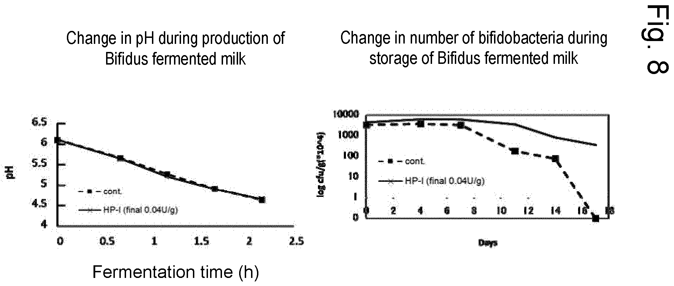

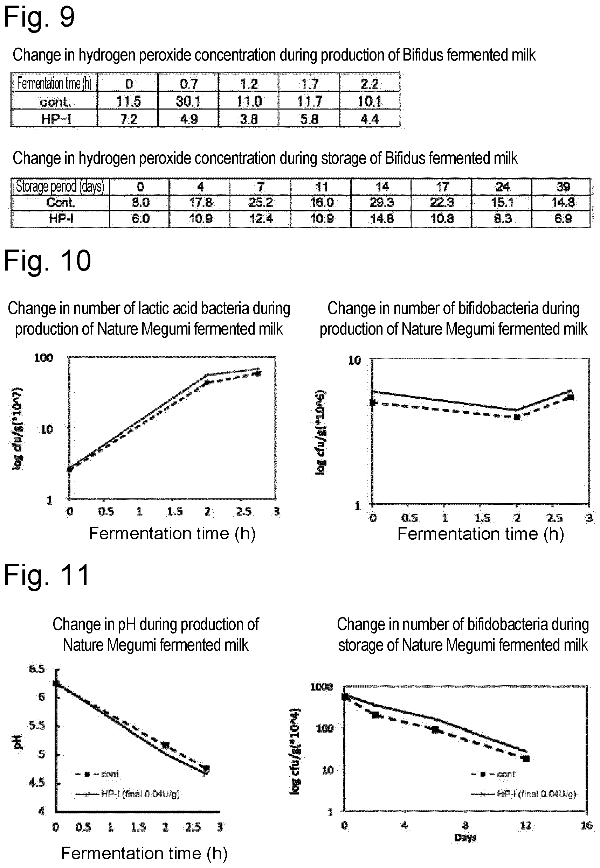

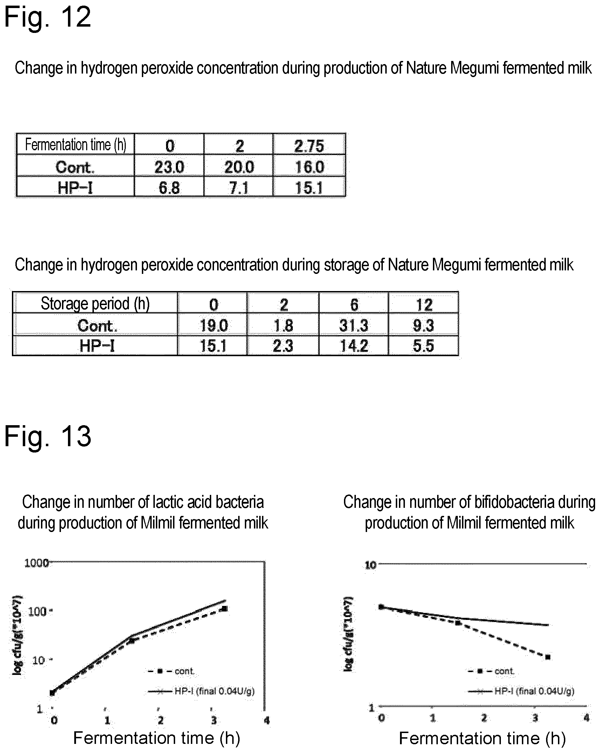

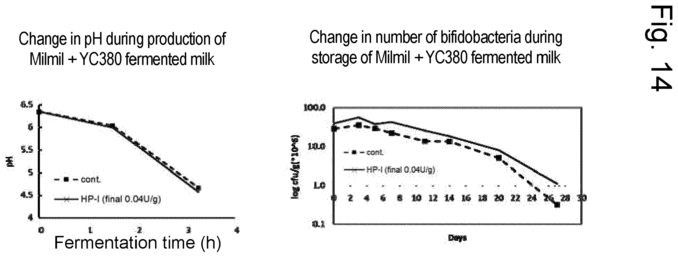

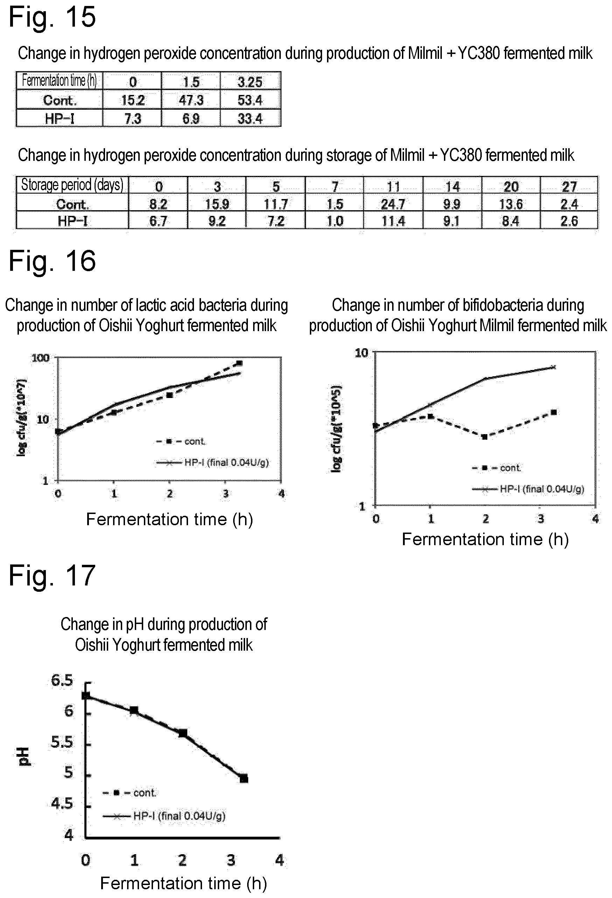

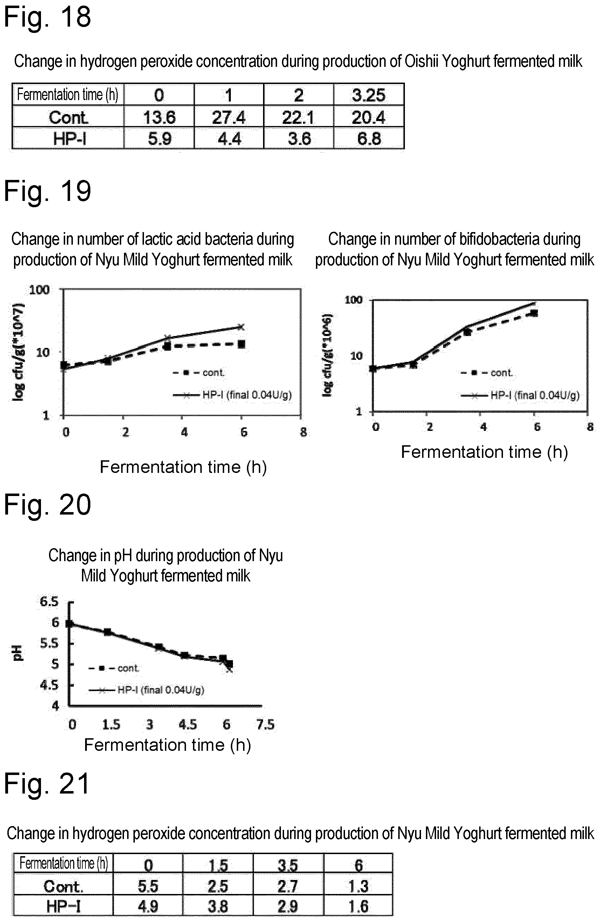

Figs. 7 to 21 show the results. The addition of the enzyme-containing composition (HP-I) after purification of Example 3 promoted the number of lactic acid bacteria and the proliferation of bifidobacteria (Figs. 7, 10, 13, 16, and 19). The pH also rapidly decreased during fermentation, and fermentation-promoting effect of about several minutes was obtained (Figs. 8, 11, 14, 17, and 20). The addition of the enzyme-containing composition suppressed the decrease rate of the number of bifidobacteria during storage of the fermented milk (Figs. 8, 11, 14, 17, and 19). It was confirmed that the hydrogen peroxide content decreased during fermentation and that generation of curds did not occur by the action of the enzyme-containing composition (Figs. 9, 12, 15, 18, and 21).

[Table 1]

| Trade name | Manufacturing company (including bifidobacteria) | Dosage %(w/w) | Note |
|---|---|---|---|
| Bifidus | MORINAGA MILK INDUSTRY CO., LTD. (Bifidobacterium longum BB536) | 6 | |
| Nature Megumi | MEGMILK SNOW BRAND Co. Ltd. (Bifidobacterium longum SBT2928) | 5 | |
| Milmil | Yakult Honsha Co., Ltd. (Bifidobacterium breve BY) | 10 | Addition of 10 mg of lactic acid bacteria YC380 (Chr. Hansen)/100 g |
| Oishii Yoghurt | KYODO MILK INDUSTRY CO., Ltd. (Maruetsu PB) (Bifidobacterium animalis subsp. lactis LKM512) | 5 | |

(continued)

| Trade name | Manufacturing company (including bifidobacteria) | Dosage %(w/w) | Note |
|---|---|---|---|
| Nyu Mild Yoghurt | NIPPON LUNA, INC. (Bifidobacterium lactis HN019) | 15 | The fermentation rate was very slow, and the pH decreased only to around 4.9, presumably because sugar, gelatin, and so on are included. |

**Claims**

1. An enzyme-containing composition having a ratio of a protease activity to a peroxidase activity of 10 or less.

2. The enzyme-containing composition according to Claim 1, wherein the ratio of a protease activity to a peroxidase activity is 2.5 or less.

3. The enzyme-containing composition according to Claim 1 or 2, wherein the ratio of a protease activity to a peroxidase activity is 0.01 or more.

4. The enzyme-containing composition according to Claim 1, comprising an enzyme having a peroxidase activity and a protease activity or comprising peroxidase and protease, wherein the ratio of a protease activity to a peroxidase activity is 0.01 or more and 10 or less.

5. The enzyme-containing composition according to Claim 1 or 2, wherein the peroxidase activity is 1 U/mL or more and 100 U/mL or less.

6. The enzyme-containing composition according to Claim 1 or 2, wherein the protease activity is 0.01 U/mL or more and 250 U/mL or less.

7. The enzyme-containing composition according to Claim 1 or 2, further comprising a stabilizing agent.

8. The enzyme-containing composition according to Claim 7, wherein the stabilizing agent is one or more selected from the group consisting of sugar alcohols, monosaccharides, oligosaccharides, and salts.

9. The enzyme-containing composition according to Claim 1 or 2, wherein the contained enzyme is at least one selected from the group consisting of catalase, peroxidase, catalase-peroxidase, manganese peroxidase, and lactoperoxidase.

10. The enzyme-containing composition according to Claim 1 or 2, wherein the contained enzyme is catalase-peroxidase.

11. The enzyme-containing composition according to Claim 10, wherein the catalase-peroxidase is an enzyme derived from an actinomycete.

12. The enzyme-containing composition according to Claim 10 or 11, wherein the catalase-peroxidase is following (a) or (b):

    (a) catalase-peroxidase composed of the amino acid sequence set forth in SEQ ID NO: 1; and
    (b) catalase-peroxidase composed of an amino acid sequence in which from one to several amino acid residues in the amino acid sequence set forth in SEQ ID NO: 1 are deleted, substituted, or inserted and having 80% or more sequence identity with the amino acid sequence set forth in SEQ ID NO: 1.

13. A method for producing milk comprising:

    a sterilization step of sterilizing raw milk; and
    a filling step of filling a container with the sterilized raw milk and then sealing the container, wherein
    the enzyme-containing composition according to Claim 1 is added to the sterilized raw milk during a period after the sterilization step and before the completion of the filling step.

**14.** A method for producing fermented milk, comprising:

a heat sterilization step of sterilizing raw milk;
an addition step of adding yeast or lactic acid bacteria to the heat-sterilized raw milk;
a fermentation step of obtaining fermented milk; and
a filling step of filling a container with the obtained fermented milk, wherein
the enzyme-containing composition according to Claim 2 is added to the sterilized raw milk during a period after
the heat sterilization step and before the completion of the filling step.

Fig. 1

# Fig. 2

(A) Peroxidase activity Final 0.05 U/mL
(B) Peroxidase activity Final 0.025 U/mL
(C) Peroxidase activity Final 0.005 U/mL

# Fig. 3

Number of lactic acid bacteria

pH

Hydrogen peroxide concentration

cont    POD (Final 0.04 U/g)

# Fig. 4

Change in number of bifidobacteria during production of YC380 + BB12 fermented milk

Change in number of lactic acid bacteria during production of YC380 + BB12 fermented milk

Change in pH during production of YC380 + BB12 fermented milk production

Change in number of bifidobacteria during storage of YC380 + BB12 fermented milk

Fig. 5

# Fig. 6

Change in hydrogen peroxide concentration during production of YC380 + BB12 fermented milk

| Fermentation time (h) | 0 | 1.5 | 2.75 | 3.25 |
|---|---|---|---|---|
| Cont. | 13.1 | 14.2 | 30.5 | 21.2 |
| HP-I | 8.5 | 6.9 | 19.2 | 22.8 |

Change in hydrogen peroxide concentration during storage of YC380 + BB12 fermented milk

| Storage period (days) | 0 | 6 | 7 | 8 | 10 | 14 | 20 |
|---|---|---|---|---|---|---|---|
| Cont. | 17.3 | 18.4 | 3.9 | 15.2 | 7.9 | 3.1 | 7.1 |
| HP-I | 10.9 | 11.2 | 3.2 | 8.3 | 3.8 | 2.8 | 3.8 |

Fig. 7

Change in number of lactic acid bacteria during Bifidus fermented milk production

Change in number of bifidobacteria during production of Bifidus fermented milk

Fermentation time (h)

Fermentation time (h)

Fig. 8

Change in number of bifidobacteria during storage of Bifidus fermented milk

Change in pH during production of Bifidus fermented milk

# Fig. 9

Change in hydrogen peroxide concentration during production of Bifidus fermented milk

| Fermentation time (h) | 0 | 0.7 | 1.2 | 1.7 | 2.2 |
|---|---|---|---|---|---|
| cont. | 11.5 | 30.1 | 11.0 | 11.7 | 10.1 |
| HP-I | 7.2 | 4.9 | 3.8 | 5.8 | 4.4 |

Change in hydrogen peroxide concentration during storage of Bifidus fermented milk

| Storage period (days) | 0 | 4 | 7 | 11 | 14 | 17 | 24 | 39 |
|---|---|---|---|---|---|---|---|---|
| Cont. | 8.0 | 17.8 | 25.2 | 16.0 | 29.3 | 22.3 | 15.1 | 14.8 |
| HP-I | 6.0 | 10.9 | 12.4 | 10.9 | 14.8 | 10.8 | 8.3 | 6.9 |

# Fig. 10

Change in number of lactic acid bacteria during production of Nature Megumi fermented milk

Change in number of bifidobacteria during production of Nature Megumi fermented milk

# Fig. 11

Change in pH during production of Nature Megumi fermented milk

Change in number of bifidobacteria during storage of Nature Megumi fermented milk

# Fig. 12

Change in hydrogen peroxide concentration during production of Nature Megumi fermented milk

| Fermentation time (h) | 0 | 2 | 2.75 |
|---|---|---|---|
| Cont. | 23.0 | 20.0 | 16.0 |
| HP-I | 6.8 | 7.1 | 15.1 |

Change in hydrogen peroxide concentration during storage of Nature Megumi fermented milk

| Storage period (h) | 0 | 2 | 6 | 12 |
|---|---|---|---|---|
| Cont. | 19.0 | 1.8 | 31.3 | 9.3 |
| HP-I | 15.1 | 2.3 | 14.2 | 5.5 |

# Fig. 13

Change in number of lactic acid bacteria during production of Milmil fermented milk

Change in number of bifidobacteria during production of Milmil fermented milk

# Fig. 14

Change in number of bifidobacteria during storage of Milmil + YC380 fermented milk

Change in pH during production of Milmil + YC380 fermented milk

# Fig. 15

Change in hydrogen peroxide concentration during production of Milmil + YC380 fermented milk

| Fermentation time (h) | 0 | 1.5 | 3.25 |
|---|---|---|---|
| Cont. | 15.2 | 47.3 | 53.4 |
| HP-I | 7.3 | 6.9 | 33.4 |

Change in hydrogen peroxide concentration during storage of Milmil + YC380 fermented milk

| Storage period (days) | 0 | 3 | 5 | 7 | 11 | 14 | 20 | 27 |
|---|---|---|---|---|---|---|---|---|
| Cont. | 8.2 | 15.9 | 11.7 | 1.5 | 24.7 | 9.9 | 13.6 | 2.4 |
| HP-I | 6.7 | 9.2 | 7.2 | 1.0 | 11.4 | 9.1 | 8.4 | 2.6 |

# Fig. 16

Change in number of lactic acid bacteria during production of Oishii Yoghurt fermented milk

Change in number of bifidobacteria during production of Oishii Yoghurt Milmil fermented milk

# Fig. 17

Change in pH during production of Oishii Yoghurt fermented milk

## Fig. 18

Change in hydrogen peroxide concentration during production of Oishii Yoghurt fermented milk

| Fermentation time (h) | 0 | 1 | 2 | 3.25 |
|---|---|---|---|---|
| Cont. | 13.6 | 27.4 | 22.1 | 20.4 |
| HP-I | 5.9 | 4.4 | 3.6 | 6.8 |

## Fig. 19

Change in number of lactic acid bacteria during production of Nyu Mild Yoghurt fermented milk

Change in number of bifidobacteria during production of Nyu Mild Yoghurt fermented milk

## Fig. 20

Change in pH during production of Nyu Mild Yoghurt fermented milk

## Fig. 21

Change in hydrogen peroxide concentration during production of Nyu Mild Yoghurt fermented milk

| Fermentation time (h) | 0 | 1.5 | 3.5 | 6 |
|---|---|---|---|---|
| Cont. | 5.5 | 2.5 | 2.7 | 1.3 |
| HP-I | 4.9 | 3.8 | 2.9 | 1.6 |

# INTERNATIONAL SEARCH REPORT

| International application No. |
| --- |
| **PCT/JP2022/028982** |

**A.    CLASSIFICATION OF SUBJECT MATTER**

*C12N 9/08*(2006.01)i; *A23C 9/13*(2006.01)i; *A23C 9/152*(2006.01)i; *C12N 9/50*(2006.01)i; *A23C 7/04*(2006.01)n
FI:    C12N9/08; A23C9/13; A23C9/152; C12N9/50; A23C7/04

According to International Patent Classification (IPC) or to both national classification and IPC

**B.    FIELDS SEARCHED**

Minimum documentation searched (classification system followed by classification symbols)

C12N9/08; A23C9/13; A23C9/152; C12N9/50; A23C7/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
| --- | --- | --- |
| X | US 4657864 A (WESTVACO CORPORATION) 14 April 1987 (1987-04-14) claims, examples | 1-9 |
| Y | | 10-12 |
| A | | 13-14 |
| X | JP 63-226243 A (AMANO PHARMA CO LTD) 20 September 1988 (1988-09-20) claims, Prior art, Problems to be solved by the invention, test examples | 1-2, 5, 7-9, 13-14 |
| Y | | 3-4, 6, 10-12 |
| Y | JP 2020-188724 A (HASEGAWA T CO LTD) 26 November 2020 (2020-11-26) claims, paragraphs [0003]-[0005], [0019], examples | 3-4, 6 |
| A | | 1-2, 5, 7-14 |
| Y | JP 2013-540447 A (VALIO LTD) 07 November 2013 (2013-11-07) claims, paragraphs [0001], [0004]-[0006] | 3-4, 6 |
| A | | 1-2, 5, 7-14 |

☑ Further documents are listed in the continuation of Box C.       ☑ See patent family annex.

| | |
| --- | --- |
| * Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **07 September 2022** | **20 September 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/028982**

**C.    DOCUMENTS CONSIDERED TO BE RELEVANT**

| Category* | Citation of document, with indication, where appropriate, of the relevant passages | Relevant to claim No. |
|---|---|---|
| Y | WO 2017/104729 A1 (GODO SHUSEI CO., LTD.) 22 June 2017 (2017-06-22)<br>  claims | 3-4, 6 |
| A |  | 1-2, 5, 7-14 |
| Y | WO 2014/185364 A1 (GODO SHUSEI CO., LTD.) 20 November 2014 (2014-11-20)<br>  paragraph [0036], examples | 3-4, 6 |
| A |  | 1-2, 5, 7-14 |
| Y | JP 2009-517061 A (DSM IP ASSETS B.V.) 30 April 2009 (2009-04-30)<br>  paragraph [0030] | 3-4, 6 |
| A |  | 1-2, 5, 7-14 |
| Y | Accession No. A0A6H0CXF6, Database UniProt [online]. 2020, [retrieval date 07 September 2022], internet: <URL: https://rest.uniprot.org/unisave/A0A6H0CXF6?format=txt&versions=1><br>  SubName, SEQUENCE | 10-12 |
| A |  | 1-9, 13-14 |
| Y | Accession No. A0A0S2PC13, Database UniProt [online]. 2016. [retrieval date 07 September 2022], internet: <URL: https://rest.uniprot.org/unisave/A0A0S2PC13?format=txt&versions=1><br>  SubName, SEQUENCE | 10-12 |
| A |  | 1-9, 13-14 |
| A | JP 62-228224 A (GLYCO KYODO NYUGYO KK) 07 October 1987 (1987-10-07) | 1-14 |
| A | JP 54-20150 A (SNAMPROGETTI SPA) 15 February 1979 (1979-02-15) | 1-14 |
| A | WO 1992/13064 A1 (SNOW BRAND MILK PRODUCTS CO., LTD.) 06 August 1992 (1992-08-06) | 1-14 |
| A | CN 111903761 A (SICHUAN DONGPO CHINESE PAOCAI INDUSTRIAL TECH RESEARCH INSTITUTE) 10 November 2020 (2020-11-10) | 1-14 |
| A | CN 102660512 A (SUZHOU KUNLAN BIOTECHNOLOGY CO LTD) 12 September 2012 (2012-09-12) | 1-14 |

Form PCT/ISA/210 (second sheet) (January 2015)

**INTERNATIONAL SEARCH REPORT**

International application No.

**PCT/JP2022/028982**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

    a.  ☑ forming part of the international application as filed:

            ☑ in the form of an Annex C/ST.25 text file.

            ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

            ☐ in the form of an Annex C/ST.25 text file (Rule 13*ter*.1(a)).

            ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    The "form of an Annex C/ST.25 text file" is replaced with the "form of ST.26."

Form PCT/ISA/210 (continuation of first sheet) (January 2015)

INTERNATIONAL SEARCH REPORT
Information on patent family members

| International application No. |
| --- |
| **PCT/JP2022/028982** |

| Patent document cited in search report | | | Publication date (day/month/year) | Patent family member(s) | | | Publication date (day/month/year) |
| --- | --- | --- | --- | --- | --- | --- | --- |
| US | 4657864 | A | 14 April 1987 | (Family: none) | | | |
| JP | 63-226243 | A | 20 September 1988 | (Family: none) | | | |
| JP | 2020-188724 | A | 26 November 2020 | (Family: none) | | | |
| JP | 2013-540447 | A | 07 November 2013 | WO claims, paragraphs [0001], [0004]-[0006] US EP CN KR | 2012/056106 2013/0230623 2632277 103188942 10-2014-0067955 | A1 A1 A1 A A | |
| WO | 2017/104729 | A1 | 22 June 2017 | US claims EP CN | 2018/0368429 3391750 108366571 | A1 A1 A | |
| WO | 2014/185364 | A1 | 20 November 2014 | US paragraph [0046], examples EP | 2016/0081361 2998395 | A1 A1 | |
| JP | 2009-517061 | A | 30 April 2009 | WO p. 8, first paragraph US EP CN | 2007/060247 2008/0286412 1954808 101316928 | A2 A1 A2 A | |
| JP | 62-228224 | A | 07 October 1987 | (Family: none) | | | |
| JP | 54-20150 | A | 15 February 1979 | GB | 1560209 | A | |
| WO | 1992/13064 | A1 | 06 August 1992 | EP | 521166 | A1 | |
| CN | 111903761 | A | 10 November 2020 | (Family: none) | | | |
| CN | 102660512 | A | 12 September 2012 | (Family: none) | | | |

Form PCT/ISA/210 (patent family annex) (January 2015)

**REFERENCES CITED IN THE DESCRIPTION**

**Patent documents cited in the description**

- JP 8004453 B